# EUROPÄISCHE PATENTSCHRIFT

(11) **EP 0 482 530 B1**
(45) Veröffentlichungstag und Bekanntmachung des Hinweises auf die Patenterteilung: **10.06.1998**
(21) Anmeldenummer: 91117840.8
(22) Anmeldetag: 18.10.1991
(51) Int. Cl.: A61K 35/74, C12N 1/04

(54) **Zusammensetzung zur Regulierung der Darmflora**
Composition for the regulation of intestinal flora
Composition pour la régulation de la flore intestinale

(30) Priorität: 25.10.1990 DE 4033996
(43) Veröffentlichungstag der Anmeldung: 29.04.1992
(73) Patentinhaber: Hölzel, Karl Heinz, D-82296 Schöngeising (DE)
(72) Erfinder: Hölzel, Karl Heinz, D-82296 Schöngeising (DE)
(74) Vertreter: Kern, Wolfgang, Dipl.-Ing.

(56) Entgegenhaltungen:
- DE-A- 2 134 179
- FR-M- 5 528
- US-A- 3 320 130
- US-A- 4 592 748
- BUNDESVERBAND DER PHARMAZEUTISCHEN INDUSTRIE E.V. 'ROTE LISTE' 1983 , EDITIO CANTOR , FRANKFURT
- Bergey's Manual of determinative Bacteriology, 1974, Seiten 670-671
- The Composition of Foods, McCance and Widdowson's, 1992, Seiten 73-77

## Beschreibung

Die Erfindung betrifft ein enzymatisches Stoffwechselprodukt zur nutritiven Substitution von lyophil getrockneten, darmresidenten, also im Darm residierenden apathogenen Reinkulturen aus Lactobacillus acidophilus und Bifidobakterium bifidum mit Nähr- und Säuerungskomponenten für die Biomasse.

Die Folgen einer reduzierten, säurebildenden Symbiontenflora (Lactobacillus acidophilus und Bifidobakterium bifidum) mit ihren Auswirkungen auf darmresidente pathogene Keime, deren Stoffwechseltoxine als Auslöser spezifischer Darmerkrankungen wie auch Störungen des Stoffwechsels bis hin zu manifestierten Stoffwechselerkrankungen, Krebs und schweren Störungen der Steuerungs- und Immunsysteme sind bekannt.

In- und ausländische Autoren sind einhellig zu den Erkenntnissen gelangt, daß eine von der biologischen Norm abweichende Besiedelung des Enddünn- und Dickdarmes mit Bifidobakterium bifidum und Lactobacillus acidophilus die zentralen Probleme der enteralen Dysbiose sind.

Die US-A-3 320 130 befaß sich bereits mit einem Medikament, das ein Lactobacillus acidophilus, Escherichia coli und Bifidobakterium bifidium enthält und zur Behandlung von Darmerkrankungen geeignet ist, wobei jedoch die in diesem Dokument genannten Primärerkrankungen, wie die genannten Coliditen als unabhängige Einzelkrankheiten angesehen werden, die auf Grund einer massiven Fehlbesiedelung eines unphysiologischen, im pH-Wert entgleisten und toxisch belasteten Intestinums ihren Ursprung haben. Um diese spezifischen Darmerkrankungen therapeutisch mit Erfolg in den Griff zu bekommen, bedarf es einer ganzheitlichen Behandlung des Dickdarms, seiner Floren und der konsequenten Wiederherstellung eines physiologischen Milieus im Intestinum mit pH-Werten unter 6.

So berichtet Perger, Wien, in einem 1985 gehaltenen Referat vor der Deutschen Gesellschaft für Thermo-Regulationstechnik, daß bei der Auswertung von 705 Stuhlkulturen von an Stoffwechselkrankheiten leidenden Patienten 54,18 % über keine Bifido- und Acidophilus-Flora mehr verfügten, während nur 41 von 705 Patienten = 5,82 % von einer eingeschränkten E.coli Besiedlung betroffen waren.

Die große Bedeutung einer biologisch nicht ausgewogenen Säuerungsflora (B.bifidum, L.acidophilus) bei Neugeborenen, Kleinkindern und älteren Kindern geht auch aus Beobachtungen in Kinderpraxen und Kinderkliniken hervor. Zunehmend, besonders aber in den letzten zwei Jahrzehnten, sehen sich Kinder- und Hautärzte sowie Allergologen bei ihren kleinen Patienten immer häufiger mit Nahrungsmittelallergie, Milchunverträglichkeit, dermatösen Affektionen, Neurodermitis, Wurm-, Pilz- und Parasitenbefall, Erkrankungen der oberen Luftwege und Nebenhöhlen und Asthma konfrontiert. Untersuchungen des faeces von an solchen Krankheiten leidenden Kleinkindern haben eine Unterbesiedlung mit B.bifidum ergeben. Der Normalablauf der Besiedelung mit züchtbaren Keimen der Intestinalflora wurde bei diesen Kindern nicht vollzogen.

Der Foetus in uteri ist steril. Die erste Kontamination findet auf dem Geburtswege statt, und zwar durch die Vaginalflora der Mutter, wobei zwangsläufig auch Anteile der perianalen Fäkalflora mit einbezogen werden. Unmittelbar nach der Geburt werden auch Keime der Umweltflora aufgenommen, wobei eine Ansiedlung solcher Organismen jedoch in der Regel unterbleibt, während die der Vaginalflora entstammenden Laktobazillen und Bifidobakterien sich rasch vermehren. Diese Besiedlung mit rasch und stark säurenden Mikroorganismen muß als Ursache dafür angenommen werden, daß sich Fäulniserreger, insbesondere Enterobakterien, nur in begrenztem Maße ansiedeln und vermehren können, da deren Wachstumsoptimum im neutralen bis leicht alkalischen Milieu liegt.

Mehrere Faktoren, warum es zur Fehlbesiedlung der Neugeborenen kommt, werden heutzutage diskutiert: Die Entbindung über den operativen Weg, eine entartete keimarme Vagina-Flora der Gebärenden durch Überhygiene oder Antibiotika-Therapie, z.B. nach Pilzbefall sowie Stillunfähigkeit oder Stillverweigerung. Neben L.acidophilus sollen die Bifidobakterien bei Stillkindern mit sogar 99 % den Hauptanteil der züchtbaren Keime im Enddünn- und Dickdarm darstellen. Neugeborene mit mangelhafter oder fehlender Säuerungsflora bleiben Problemkinder. Unter Normverhältnissen begleiten das Bifidobakterium bidifum den Menschen ein Leben lang.

Die Erfindung stellt sich bei diesem dramatischen Verlauf der enteralen Dysbiose die Aufgabe, eine sogenannte Symbioselenkung durchzuführen und in diesem Zusammenhang das enzymatische Stoffwechselprodukt der eingangs genannten Art so auszubauen, daß sichergestellt wird, daß geburtsbedingte, biologisch nicht ausgewogene bzw. stark dezimierte oder total eliminierte Säuerungsfloren im Intestinum regeneriert bzw. neu angesiedelt werden.

Diese Aufgabe wird erfindungsgemäß dadurch gelöst, daß die Nähr- und Säuerungskomponenten sich zusammensetzen aus Milchserum, Rote Bete und Zitronensäure, wobei die genannten Substanzen in enddünndarmlöslichen Hartgelatinekapseln eingeschlossen sind, so daß die Biomasse durch eigene Stoffwechselaktivitäten, wobei das Stoffwechselprodukt die L-(+)Milchsäure ist, den für ein physiologisches Darmmilieu notwendigen pH-Wert unter 6 erbringt.

Hierdurch wird gewährleistet, daß Entgiftungs-, Resorptions-, Assimilations- und Stoffwechselfunktionen des Darms wieder in einem ausreichenden Maße erfüllt und die immunologischen Abläufe sowie die Selbstheilungskräfte aktiviert werden, so daß Obstipationen, colon irritabile, Völlegefühl, Gasbildung, Flatulenz, Enzymblockaden, Überbelastung der Leber und infolge Müdigkeit sowie die durch die Toxinlage erfolgende Auslösung stoffwechselabhängiger Zivilisationskrankheiten, Pilzbefall durch Immunschwäche mit ihren gefürchteten Komplikationen vermieden werden.

Die orale Substitution mit erfindungsgemäßen lyophilen L-(+) Sub-Kulturen geht gegenüber der herkömmlichen mikro-biologischen Therapie, die durch E.coli-Substitution als Antigen auf eine Immunreaktion (Immunantwort) abzielt, einen anderen Weg. Die Substitution von E.coli z.B. bei rezidivierenden Infekten kann über die Dauer der E.coli-Gaben zwar Immunreaktionen des latenten Immunsystems auslösen, muß aber als sogenannte Symptomtherapie angesehen werden. Das auslösende Moment der Immunschwäche, das unphysiologische mit enteralen Toxinen belastete Dickdarmmilieu, läßt die Implantation einer neuen Säuerungsflora ins Colon unberücksichtigt.

Auf dem Markt befindliche Kapselpräparate mit säuernden Lactobacillus acidophilus und Bifidobakterium bifidum haben kein Nährsupplement und keine Substanzen, die im Dünn- und Dickdarmmilieu ein Lebensoptimum - pH- und Nährmilieu - für implantierte Mikroben anbieten.

Die erfindungsgemäßen essentiellen Nähr- und Säuerungskomponenten für die mikrobielle Biomasse sind durch mikrofeine Zerkleinerung bzw. Zerlegung der Bestandteile der Nähr- und Säuerungskomponenten mittels einer oder mehrerer bekannter Desintegratoren behandelt, die auf das Massengemisch mechanische Schläge in verschiedenen Schlagfolgen ausüben und zwar vorteilhafterweise bei gleichzeitiger Einwirkung eines statischen und/oder dynamischen Magnetfeldes

Eine derartige Behandlung ist zur mechanischen Wasserbehandlung bekannt geworden (DE-OS 30 04 664), dient jedoch dort zur Steuerung der Eigenschaften des Wassers durch Beeinflussung der molekularen Struktur durch Frequenzeinwirkung mittels mechanischer Schläge, vorzugsweise von definierter Stärke, Zahl- und Zeitfolge, um das Wasser zum Zwecke der Steigerung seiner Lösungsfähigkeit für ohne spezielle Zusätze schwer bis nicht lösbare oder emulgierbare Substanzen zu beeinflussen. Dabei ist daran gedacht, die Eigenschaft des Wassers als Wasch- und Reinigungsmittel auch ohne oder mit wesentlich verminderten Zugaben von Netzmitteln und konditionierenden Mitteln zu verbessern, insbesondere für Fette aufnahmefähiger zu machen.

Ein neuerer Vorschlag verfolgt eine andere Richtung, indem die Schlagwirkung des oder der Desintegratoren eine mikrofeine Zerkleinerung und damit einen entsprechenden Aufschluß der Substanzen bewirken soll, aus denen die Nähr- und Säuerungsmasse zusammengesetzt ist, um die Wirksamkeit dieser Substanzen zu erhöhen und ihre gegenseitige Reagenz zu verstärken und dadurch mittels dieser mechanischen Aktivierung die Stoffwechselaktivitäten der mikrobiellen Biomasse zu aktivieren, die durch die Eigenproduktion von L-(+)-Milchsäure sich ein optimales Milieu zur Ansiedlung und Weitervermehrung schafft.

Untersuchungen an Zerkleinerungsvorgängen haben gezeigt, daß die aufgewandte Energie bei bestimmten hochenergetischen Verfahren größer ist als die Summe aus Zerkleinerungsarbeit und freigesetzter Wärme. Die Differenz entspricht der Aktivierungsenergie. Sie ist im Mahlgut gespeichert und kann für verschiedene chemische und physikochemische Folgereaktionen als Auslöser oder Reaktionsverbesserung nutzbar gemacht werden, indem sie den Schwellenwert der betreffenden Reaktionen herabsetzt bzw. diese beschleunigt.

Bei der Bearbeitung des Massengemisches in einer Desintegrationsanlage vollziehen sich mehrere entscheidende meßbare Phänomene, die im folgenden erläutert werden.

In einem homöopathischen Mono-Arzneimittel, welches z.B. Bestandteile von alkoholischen Lösungen bzw. Auszüge aus Heilpflanzen in potenzierten Verdünnungen mit Aethanol (z.B. D 1, D 6, D 12, D 30, D 200) nach der Hahnemann'schen Lehre enthält, sind in den hohen Potenzen die pflanzlichen Anteile kaum noch analysierbar.

Durch das Prinzip der mechanischen oder manuellen Verschüttelung der in alkoholischer Basis gelösten Auszüge werden deren pflanzliche Informationen (Botenstoffe) bis in die hohen Potenzen weitergegeben und bleiben dort verfügbar.

Dies gelang bisher nur im alkoholischen Milieu. Durch das Prinzip der Verschüttelung entsteht zur Information ein meßbares bio-energetisches Terrain (Microstrahlungen/Schwingungen). Information und begrenzte bio-energetische Schwingungen als Auswirkung des Verschüttelungseffektes stimulieren Regelmechanismen der Organe und Zellaktivität.

Problematisch bleibt für Kleinkinder, Jugendliche, Antialkoholiker, Alkoholgefährdete, gereatrische Menschen der in den homöopathischen Arzneimitteln bis zu 64 Vol.% vorhandene Alkohol.

Durch das erfindungsgemäße Herstellungsverfahren mit der Desintegrationseinrichtung werden in der Relation zur voluminösen hydrophilen Phase ebenfalls relativ geringfügige Quantitäten an natürlichen Heilpflanzen und anderen Zutaten verwendet.

Während bei den homöopathischen Drogenauszügen die vom menschlichen Organismus benötigten wertvollen pflanzlichen Eiweiß- und Mineralienbausteine der Pflanzen nicht voll vorhanden sind, verbleiben diese Wirkstoffe durch die mikrofeine Dispersion der ganzen Pflanzen vollständig im enzymatischen Stoffwechselprodukt.

Durch die Einwirkung der hohen Schlagzahl der rotierenden Schlagelemente und eines statischen oder dynamischen und/oder elektrischen Spannungsfeldes gelingt es erstmalig, trotz der quantitativ geringen vegetabilen Vitalstoffanteile deren Information (Botenstoffe) in eine hydrophile Phase zu integrieren, was mit bio-energetischen Messungen festgestellt werden konnte.

Bio-energetische Terrain-Vergleichsmessungen zwischen homöopathischen Arzneimitteln und dem hydrophilen enzymatischen Stoffwechselprodukt haben ein bis zu 180 bis 200 % höheres bio-energetisches Schwingungsfeld aufgezeigt.

In der Anwendungspraxis bedeutet ein höheres bio-energetisches Schwingungsfeld, wobei die im Massengemisch des enzymatischen Stoffwechselproduktes durch Beimpfung mit Kulturen erzeugte L-(+) Milchsäure auch als Adapter tätig wird, eine spezifische optimale Resorption und Assimilation der bioverfügbaren Information bzw. der Botenstoffe zur Stimulierung der teilweise blockierten oder fehlgesteuerten Regelsysteme zu regenerierenden Zellaktivitäten und Auslösung der Selbstheilungskräfte. Das Problem Alkohol ist nicht mehr relevant.

Die Trägerphase der Information bzw. der Botenstoffe, nämlich Trinkwasser oder Brunnenwasser, so haben Messungen ergeben, besitzt so gut wie kein bio-energetisches Terrain.

## Patentansprüche

1. Enzymatisches Stoffwechselprodukt zur nutritiven Substitution von lyophil getrockneten, darmresidenten, apathogenen Biomassen aus Lactobacillus acidophilus und Bifidobakterium bifidum mit Nähr- und Säuerungskomponenten für die Biomasse, **dadurch gekennzeichnet,**daß die Nähr- und Säuerungskomponenten sich zusammensetzen aus Milcherserum, Rote Bete und Zitronensäure, wobei die genannten Substanzen in enddünndarmlöslichen Hartgelatinekapseln eingeschlossen sind, so daß die Biomasse durch eigene Stoffwechselaktivitäten, wobei das Stoffwechselprodukt die L-(+)Milchsäure ist, den für ein physiologisches Darmmilieu notwendigen pH-Wert unter 6 erbringt.

## Claims

1. Enzymatical metobolite for the nutritive substitution of lyophilic, dried intestine resistent, apathogene biomasses of lactobacillus acidophilus and bifidobacterium bifidum with nutritive and acidifying components for the biomass, characterized in that the nutritive and acidifying components are composed of lactic serum, red beet and citric acid, wherein the said substances are enclosed in hard capsules of gelatine soluble within the small intestine so that the biomass produces by metabolism activities of its own the pH-value below 6 wich is necessary for a physiological enviroment of the intestine, wherein the metobolite comprising is the L-(+) lactic acid.

## Revendications

1. Produit métabolique enzymatique pour la substitution nutritive de biomasses apathogènes, séchées par lyophilisation, résidant dans l'intestin, de Lactobacillus acidophilus et de Bifidobacterium bifidum par des composants de nutrition et d'acidification pour la biomasse, caractérisé en ce que les composants de nutrition et d'acidification sont constitués de sérum lactique, de betterave rouge et d'acide citrique, les substances mentionnées étant enfermées dans des capsules de gélatine dure solubles dans l'intestin grêle terminal de telle sorte que la biomasse, du fait de ses activités métaboliques propres, où le produit métabolique est l'acide lactique L-(+), apporte le pH inférieur à 6 nécessaire à un milieu intestinal physiologique.
